# EUROPÄISCHE PATENTSCHRIFT

(11) **EP 2 498 728 B1**
(45) Veröffentlichungstag und Bekanntmachung des Hinweises auf die Patenterteilung: **16.04.2014**
(21) Anmeldenummer: 10782545.7
(22) Anmeldetag: 12.11.2010
(51) Int. Cl.: A61F 2/68, G08C 17/00, A61F 5/01, A61F 2/64

(54) **SYSTEM MIT ZUMINDEST EINER ORTHOPÄDIETECHNISCHEN EINRICHTUNG UND EINER FERNBEDIENUNG**
SYSTEM WITH AT LEAST ONE ORTHOPAEDIC DEVICE AND REMOTE CONTROL
SYSTÈME COMPRENANT AU MOINS UN DISPOSITIF ORTHOPÉDIQUE ET UNE TÉLÉCOMMANDE

(30) Priorität: 13.11.2009 DE 102009052891
(43) Veröffentlichungstag der Anmeldung: 19.09.2012
(73) Patentinhaber: Otto Bock Healthcare Products GmbH, 1070 Wien (AT)
(72) Erfinder: POP, Constantin, A-2630 Ternitz (AT)
(74) Vertreter: Stornebel, Kai
(86) Internationale Anmeldenummer: PCT/EP2010/006890
(87) Internationale Veröffentlichungsnummer: WO 2011/057789

(56) Entgegenhaltungen:
- EP-A2- 1 981 012
- DE-A1- 10 351 916
- GB-A- 2 280 609
- US-A1- 2009 016 728

## Beschreibung

Die Erfindung betrifft ein System mit zumindest einer orthopädietechnischen Einrichtung mit zumindest einem Gelenk und einer dem jeweiligen Gelenk zugeordneten Widerstandseinrichtung, der eine Steuerungseinrichtung und ein Aktuator zugeordnet sind, wobei über den Aktuator das Gelenk bewegt oder der Widerstand gegen eine Beuge- und/oder Streckbewegung veränderbar ist, sowie mit einer Fernbedienung, die mit der Steuerungseinrichtung gekoppelt ist und über die das Widerstandsverhalten veränderbar ist. Überwiegend werden solche Antriebe oder elektronisch verstellbaren Widerstandseinrichtungen in orthopädietechnischen Einrichtungen der unteren Extremitäten eingesetzt, insbesondere bei Beinprothesen oder Beinorthesen, wobei die einstellbaren Widerstandseinrichtungen vorwiegend bei Prothesenkniegelenken, Prothesenhüftgelenken sowie Prothesenfußgelenken eingesetzt werden. Grundsätzlich ist es jedoch auch vorgesehen, neben dem Einsatz in prothetischen und orthetischen Einrichtungen der unteren Extremitäten auch Prothesen oder Orthesen an oberen Extremitäten mit entsprechenden Systemen auszurüsten.

Aus der DE 103 51 916 A1 ist ein Prothesenkniegelenk mit einem Oberteil und einem Unterteil bekannt, die miteinander durch eine mehrachsige Gelenkeinrichtung schwenkbar verbunden sind. Zwischen dem Oberteil und dem Unterteil ist eine Widerstandseinrichtung angeordnet, die der Beugung entgegenwirkt. In einer Ausgestaltungsform ist eine Arretiereinrichtung zum Sperren des Prothesenkniegelenkes in gestreckter Stellung vorgesehen, wobei die Arretiereinrichtung durch eine Betätigungseinrichtung verriegelbar und entriegelbar ist. Zur leichteren Bedienbarkeit ist eine Betätigungseinrichtung vorgesehen, die über eine Fernbedienung angesteuert ist.

Die DE 103 11 189 B4 beschreibt ein orthopädietechnisches Hilfsmittel mit zwei relativ zueinander bewegbaren Teilen und einer Verriegelungsvorrichtung zum Verriegeln der beiden Teile in einer vorbestimmten relativen Position sowie zum Entriegeln der Teile und zur Freigabe der Bewegung der Teile zueinander. Die Betätigung der Verriegelungsvorrichtung kann mittels einer drahtlosen Übertragung eines Betätigungssignals erfolgen.

Die DE 600 15 348 T2 beschreibt eine die Existenz oder die Funktion einer Gliedmaße ersetzenden Unterstützungsvorrichtung aus mindestens zwei, durch ein künstliches Gelenk miteinander verbundenen Teilen und einer Kontrollvorrichtung für das Gelenk. Die Kontrollvorrichtung ist so angeordnet, dass sie das Gelenk auf der Basis von Neigungswinkeldaten beeinflusst. Vor dem Hinsetzen kann das Kniegelenk durch eine spezielle Funktion, beispielsweise manuell durch ein Steuerungsmittel wie z.B. einen am Körper oder an der Unterstützungsvorrichtung aufgebrachten Druckknopf, freigegeben werden. Das Kniegelenk bleibt freigegeben, bis der Sensor anzeigt, dass ein Winkel bis zu einem vorbestimmten Grenzwert abgenommen hat.

Die US 2009/0016728 A1 betrifft ein Verfahren und ein System, bei dem eine Fernbedienung mit einer Vorrichtung über ein Pairing-Verfahren miteinander gekoppelt wird. Dazu wird die Fernbedienung in einen Reprogrammierungsmodus versetzt und ein Originalidentifizierer in einen davon abweichenden Identifizierer innerhalb der Fernbedienung verändert, so dass alle Steuerungssignale von der Fernbedienung den neuen Identifizierer aufweisen. Anschließend wird die Signalstärke der Fernbedienung wesentlich verringert, was den Nutzer dazu veranlasst, die Fernbedienung sehr nah an die Vorrichtung heranzuführen, um ein besonderes Kommando zu empfangen, das den neuen Identifizierer der Fernbedienung aufweist, wobei die Vorrichtung nachfolgend nur auf Steuerungssignale der Fernbedienung reagiert, die den neuen Identifizierer aufweisen.

Die EP 1 981 012 A2 betrifft eine Fernbedienung, eine elektronische Einrichtung sowie ein Fernsteuersystem, bei dem ein Pairing-Prozess über eine Zweiwegekommunikation aufgebaut wird. Eine lichtaussendende Einheit übermittelt einen Anfragecode unter Verwendung von Infrarotstrahlung, um die Installation des Pairing-Prozesses zu beginnen. Eine Kommunikationseinheit übermittelt eindeutige Informationen des elektronischen Gerätes unter Verwendung von Radiowellen als Antwort der Anfrage der lichtemittierenden Einheit und versendet als Antwort Radiowellen. Eine Steuerungseinheit steuert die lichtemittierende Einheit und die Kommunikationseinheit, um die eindeutige Information aufzunehmen, die durch die Kommunikationseinheit empfangen wurde und legt den Partner des Pairing-Prozesses fest.

Die GB 2 280 609 A betrifft ein adaptives Prothesensteuerungssystem für ein Kunstglied mit einer Steuerungsvorrichtung für die Bewegung, einem Sensor zur Erzeugung eines elektrischen Signals in Abhängigkeit von der Bewegung, einer elektronischen Verarbeitungseinrichtung, die mit der Steuerung und dem Sensor gekoppelt ist sowie einer Fernbedienungssteuerungseinheit für die Übermittlung eines Steuerungssignals für das Kunstglied. Ein Empfänger bildet einen Teil des Kunstgliedes und ist mit der Verarbeitungseinrichtungt gekoppelt.

Aufgabe der vorliegenden Erfindung ist es, ein System bereitzustellen, mit dem es möglich ist, eine sichere und flexible Anpassung einer orthopädietechnischen Einrichtung an die Bedürfnisse der Nutzer vornehmen zu können.

Erfindungsgemäß wird diese Aufgabe durch ein System mit den Merkmalen des Hauptanspruches gelöst. Vorteilhafte Ausgestaltungen und Weiterbildungen der Erfindung sind in den Unteransprüchen aufgeführt.

Das erfindungsgemäße System mit zumindest einer orthopädietechnischen Einrichtung mit zumindest einem Gelenk und einer dem jeweiligen Gelenk zugeordneten Widerstandseinrichtung, der eine Steuerungseinrichtung und ein Aktuator zugeordnet sind, wobei über den Aktuator das Gelenk angetrieben oder der Widerstand gegen eine Beuge- und/oder Streckbewegung veränderbar ist, sowie mit einer Fernbedienung, die mit der Steuerungseinrichtung gekoppelt ist und über die das Widerstandsverhalten veränderbar ist, sieht vor, dass in der orthopädietechnischen Einrichtung Daten für die Fernbedienung abgelegt sind, mit denen die Fernbedienung konfigurierbar ist und die über einen Pairing-Prozess auf die Fernbedienung übertragen werden. In der orthopädietechnischen Einrichtung, und dort insbesondere in der Steuerungseinrichtung, sind Daten gespeichert, die z.B. die Parameter oder Steuerungsmodi betreffen, wobei die Daten für die Fernbedienung vorgesehen sind und die Fernbedienung über einen Pairing-Prozess mit der Steuerungseinrichtung koppelbar ist. Dadurch ist es möglich, dass die Fernbedienung keine Voreinstellung hinsichtlich der jeweiligen Parameter oder Steuerungsmodi bedarf, sondern dass die einzustellenden

Parameter, Kennfelder oder auch Benutzeroberflächen der jeweiligen Steuerungseinrichtung zugeordnet auf die Fernbedienung übertragen werden. Die Fernbedienung und die orthopädietechnische Einrichtung werden dabei aufeinander abgestimmt. Jedes Gelenk mit der jeweils zugeordneten Steuereinrichtung wird mit der Fernbedienung gekoppelt und darauf abgestimmt, so dass unterschiedliche Daten oder Steuerungsprogramme für die jeweiligen Gelenke oder Steuerungseinrichtungen individuell auf die genutzte Fernbedienung übertragbar sind. Dadurch ist es nicht notwendig, spezielle Fernbedienungen vorzusehen, ggf. sogar mehrere Fernbedienungen für eine orthopädietechnische Einrichtung vorzusehen, vielmehr ist es möglich, mit einer nur grundsätzlich vorgerüsteten Fernbedienung eine Vielzahl an ggf. auch unterschiedlichen orthopädietechnischen Einrichtungen steuern oder einstellen zu können. Über die Fernbedienung kann dann beispielsweise der Zeitpunkt der Erhöhung oder Verringerung von Widerständen und/oder eine Erhöhung oder Verringerung von Widerständen als solche individuell auf die Bedürfnisse des jeweiligen Nutzers eingestellt werden. Auch können Betriebsmodi aktiviert oder deaktiviert werden. Dabei ist es selbstverständlich, dass die Fernbedienung auch eine Sendeeinrichtung zur Übertragung von Signalen in die orthopädietechnische Einrichtung aufweist und diese wiederum die Informationen oder Signale mit einer dafür vorgesehenen Empfangseinrichtung aufnimmt. Zur einfachen Übertragung der Daten von der orthopädietechnischen Einrichtung, beispielsweise der Prothese oder Orthese, auf die Fernbedienung, die lediglich mit einer Grundstruktur und Grundfunktionalität ausgestattet ist, ist vorgesehen, dass in der orthopädietechnischen Einrichtung ein Sender und in der Fernbedienung ein Empfänger angeordnet ist, über die drahtlos eine Datenübertragung stattfinden kann. Als Zusatzmöglichkeit zur Datenübertragung können Schnittstellen für Datenleitungen vorgesehen sein, so dass die Daten über eine physische Verbindung zwischen der Fernbedienung und der orthopädietechnischen Einrichtung übertragen werden können. Die Schnittstellen, üblicherweise Buchsen oder Stecker, können alternativ oder zusätzlich zu dem Sender und dem Empfänger vorhanden sein.

In der Steuerungseinrichtung können verschiedene Steuerungsmodi abgelegt sein, die über die Fernbedienung aktivierbar oder deaktivierbar sind. Jede Steuerung einer orthopädietechnischen Einrichtung mit einem Gelenk weist eine Grundfunktionalität auf, mit der es möglich ist, ein Standardbewegungsprogramm auszuführen. Bei Knieprothesen oder Knieorthesen sind dies beispielsweise das Gehen in der Ebene und das Gehen auf geneigten Ebenen. Sollen zusätzliche Möglichkeiten bereitgestellt werden, um eine angepasste Steuerung und ein angepasstes Antriebs- oder Widerstandsverhalten zu erhalten, werden Zusatzmodi aktiviert, die bevorzugt in der Steuereinrichtung in der orthopädietechnischen Einrichtung abgelegt sind. Dadurch ist es möglich eine orthopädietechnische Einrichtung mit einem potentiell vollständigen Programmumfang bereitzustellen, von dem jedoch nur Teile freigeschaltet sind. Die Freischaltung erfolgt dabei zum Beispiel auf der Grundlage physiologischer oder therapeutischer Erkenntnisse, so dass es möglich ist, durch eine unterschiedliche Freischaltung von Modi einen unterschiedlichen Leistungsumfang zu realisieren. Durch die Hinterlegung der Modi in den jeweiligen Steuereinrichtungen in den Gelenken ist es möglich, angepasste Steuerungsprogramme den jeweiligen Gelenken zuzuordnen, so dass es nicht notwendig ist, eine Fernbedienung mit sämtlichen möglichen Steuerungsmodi ausstatten zu müssen.

In der orthopädietechnischen Einrichtung sind vorzugsweise Daten gespeichert, die Steuerungsparameter, Steuerungsmodi, Sprache, Anordnung von Schaltflächen, Tastenbelegungen und /oder Einstellumfänge betreffen, so dass es möglich ist, die Fernbedienung mit der und auf die orthopädietechnische Einrichtung zu konfigurieren. Die dazu benötigten Daten sind auf der orthopädietechnischen Einrichtung abgelegt und werden auf die Fernbedienung übertragen. Nach dem Pairing-Prozess, bei dem die Fernbedienung auf die Orthese oder Prothese abgestimmt wird, weist die Fernbedienung das von ihr benötigte Datenmaterial auf, um die Prothese oder Orthese im Rahmen der eingeräumten Rechte zur Verstellung einstellen zu können. Auf der Orthese oder Prothese kann abgelegt sein, in welcher Reihenfolge auf der Fernbedienung welcher Modus erscheint, in welchem Rahmen Parameter der Steuerung verstellt werden können, wie viele Modi verstellbar sind, ob Funktionen freischaltbar sind und welche Tasten welche Funktion haben. Unter einem Modus wird ein Steuerungsverhalten verstanden, das willkürlich ein- und ausschaltbar ist, während eine Funktion grundsätzlich vorhanden ist und insbesondere sicherheitsrelevante Abläufe steuert und die Grundfunktionalität absichert. Über die übertragenen Daten werden auch die Verstellmöglichkeiten definiert, also wie weit die Verstellung durch den Nutzer der Fernbedienung gehen darf. Die Rechte zur Verstellung sind gegenüber einem Orthopädietechniker begrenzt, der die Steuerung in der Regel über eine gesonderte Schnittstelle verändert.

Über diese gesonderte Schnittstelle kann der Orthopädietechniker dann zum Beispiel auch die Daten, die später auf die Fernbedienung übertragen werden, konfigurieren. So kann er zum Beispiel die Art, Reihenfolge, Namen und Anzahl der Zusatzmodi definieren und die Menüsprache für die Fernbedienung in die orthopädietechnische Einrichtung übertragen.

Weiterhin ist vorgesehen, dass die Fernbedienung in der Lage ist, mehrere, ggf. unterschiedliche Steuerungseinrichtungen, Gelenke oder auch orthopädietechnische Einrichtungen insgesamt anzusprechen. Es ist möglich, dass unterschiedliche Gelenkeinrichtungen an einem Nutzer oder Patienten angesteuert werden, ebenso können gleichartige Einrichtungen an verschiedenen Patienten angesprochen werden. Gelenkeinrichtungen für Knie, Füße, Hände, Ellenbogen oder Hüften an einer Person sind ebenso ansteuerbar wie mehrere Gelenkeinrichtungen, die jeweils nur einem Gelenktyp zuzuordnen sind. Dies ist insbesondere für Orthopädietechniker von Vorteil, da mit lediglich einer Fernbedienung eine Vielzahl von Patienten mit einer Vielzahl unterschiedlicher orthopädietechnischer Einrichtungen betreut werden kann. Ist ein Steuerungsmodul für mehrere Gelenkeinrichtungen in einer orthopädietechnischen Einrichtung vorgesehen, beispielsweise in Gestalt einer zentralen Recheneinheit, kann die Zuordnung innerhalb der Fernbedienung leicht über eine Kennung erfolgen. Bei mehreren Steuerungseinrichtungen ist vorteilhafterweise vorgesehen, dass die Steuerungseinrichtungen an einer orthopädietechnischen Einrichtung eine gemeinsame Grundkennung aufweisen, die um eine Kennung für das jeweilige Gelenk bzw. die jeweilige Steuerungseinrichtung oder das jeweilige Steuerungsprogramm ergänzt wird. Die Fernbedienung kann dazu ausgebildet sein, zwischen mehreren Steuerungsmodi umzuschalten und/oder Steuerungsmodi freizuschalten, wobei das Freischalten bedeutet, dass ein bestimmter Modus aktiviert wird, wenn beispielsweise über Sensoren bestimmte Rahmenbedingungen oder Bewegungszustände detektiert werden. Ein Umschalten bedeutet, dass das jeweils angewählte Programm oder der angewählte Modus gegenwärtig vorhanden ist und ausgeführt werden muss. Das Aktivieren oder Deaktivieren bewirkt eine Wirksamkeit des jeweiligen Modus.

Über die Fernbedienung ist es möglich, die Steuerungseinrichtung in einen Tiefschlafmodus zu schalten und sie dann ggf. aus diesem Tiefschlafmodus herauszuholen. Die Steuerungseinrichtung kann z.B. in dem Tiefschlafmodus zyklisch hochfahren und prüfen, ob von der Fernbedienung neue Signale vorliegen. Ist dies der Fall, wird der Tiefschlafmodus beendet und die Steuerungseinrichtung voll aktiviert. Ebenfalls ist es möglich, dass die Fernbedienung eine zyklische Anfrage an die Steuerungseinrichtung sendet, ob sich Änderungen in der orthopädietechnischen Einrichtung ergeben haben. Sollte dies der Fall sein, wird der Tiefschlafmodus beendet und die Steuerungseinrichtung voll aktiviert.

Eine Weiterbildung der Erfindung sieht vor, dass der Fernbedienung eine Nutzerkennung zugeordnet ist, über die definiert ist, welche Parameter und/oder in welchem Umfang Parameter nutzerabhängig verstellbar sind. Jeder Nutzer erhält eine Kennung. Die Nutzerkennung ermöglicht es, dass der jeweilige Nutzer einer Fernbedienung mit definierten Rechten ausgestattet ist, welche Parameter der Steuerung für die Widerstandseinrichtung geändert werden können und welche nicht. Ebenfalls ist es vorgesehen, dass Rechte hinsichtlich des Änderungsumfanges der Parameter dem jeweiligen Nutzer zugeordnet werden, so das eine nutzerabhängige Einstellung der Parameter und damit auch der Steuerungsprogramme erfolgen kann. Es ist beispielsweise möglich, dass ein Orthopädietechniker wesentlich umfangreichere Änderungsmöglichkeiten in der Steuerung erhält als der Nutzer der orthopädietechnischen Einrichtung, da davon ausgegangen werden kann, dass ein Orthopädietechniker aufgrund seiner umfangreichen Ausbildung notwendige Änderungen zur Anpassung des Steuerungsverhaltens in einem größeren Umfang durchführen kann als der Endbenutzer. Solche grundlegenden Änderungen können aufgrund von Bewegungsuntersuchungen und Bewegungsbeobachtungen erfolgen, ebenfalls können veränderte Steuerungsmodi an den sich verändernden Bewegungsablauf, beispielsweise im Rahmen von Heilungsprozessen oder Adaptionsprozessen, angepasst werden. Der Endnutzer hat dagegen nur eingeschränkte Verstellmöglichkeiten über die Fernbedienung, um die Steuerung an die individuellen Bedürfnisse anpassen zu können.

Die Nutzerkennung kann beispielsweise als ein eingebbarer Code ausgebildet sein, beispielsweise ein numerischer Code, ein alphanumerischer Code oder ein anderer Code. Der individuelle Nutzer erhält dabei einen anderen Code als beispielsweise der Orthopädietechniker oder der Programmierer innerhalb des die orthopädietechnische Einrichtung herstellenden Unternehmens. Über den Code kann dann im Pairing-Prozess festgestellt werden, welche Rechte dem jeweiligen Nutzer der Fernbedienung eingeräumt worden sind, so dass in dem vorgegebenen Rahmen hinsichtlich der Art und des Umfanges der einzustellenden Parameter die Einstellbarkeit freigegeben wird. Alternativ oder ergänzend ist vorgesehen dass die Fernbedienung einer Fassungseinrichtung für biometrische Daten aufweist, über die eine Nutzererkennung zugeordnet wird. Die biometrische Datenerkennungseinrichtung kann beispielsweise ein Fingerabdruckscanner sein, ebenfalls können andere biometrische Daten in der Fernbedienung hinterlegt werden, um eine individuelle Zuordnung zu erreichen.

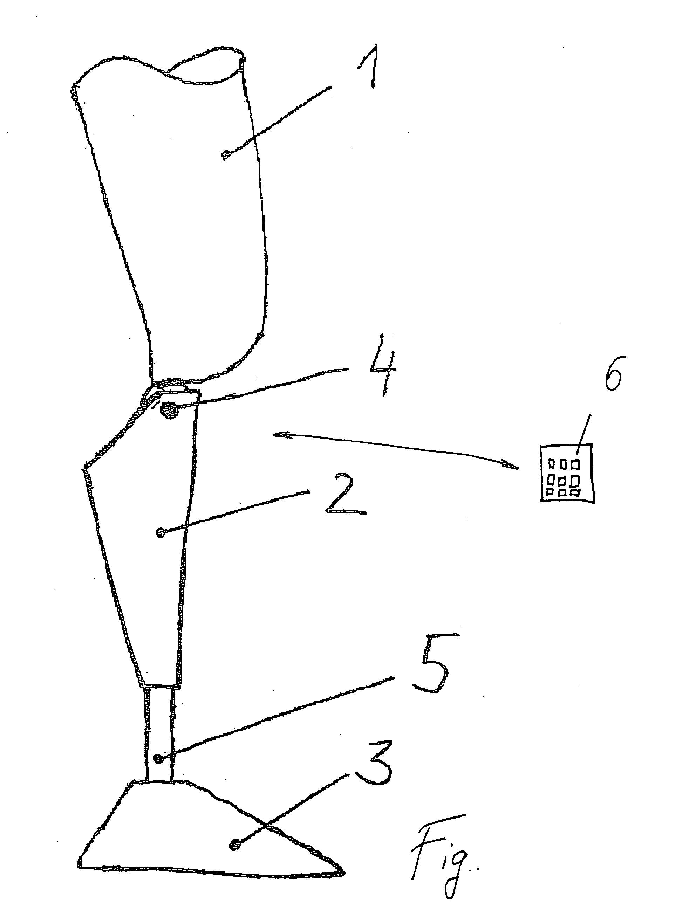

Nachfolgend wird ein Ausführungsbeispiel der Erfindung anhand der beigefügten Figur näher erläutert. Die einzige Figur zeigt eine Prothese mit einem Oberschenkelschaft 1 und einem Unterschenkelschaft 2, die über ein Prothesenkniegelenk 4 gelenkig miteinander verbunden sind. In dem Unterschenkelschaft 2 ist eine Steuerungseinrichtung für ein ebenfalls in dem Unterschenkelschaft 2 angeordnete, nicht dargestellte Widerstandseinrichtung untergebracht. Ein Prothesenfuß 4 ist an dem distalen Ende des Unterschenkelteils 2 angebracht und über einen Verbindungsschaft 5 mit dem Unterschenkelteil 2 verbunden.

Die nicht dargestellte Steuerungseinrichtung ist über eine bidirektionale Kommunikationsverbindung, beispielsweise über eine Funkstrecke oder über ein Kabel, mit einer Fernbedienung 6 verbunden. Die Fernbedienung 6 kann eine Tastatur aufweisen, ebenfalls ist es möglich, dass die Fernbedienung 6 als Touchscreen ausgebildet ist. Über einen Pairing-Prozess wird die Fernbedienung 6 mit der Steuerungseinrichtung in dem Unterschenkelteil 2 gekoppelt. In der Steuerungseinrichtung sind beispielsweise Daten über die Grundfunktionen der Steuerung für das Prothesenkniegelenk 4 abgelegt. Darüber hinaus sind Steuerungsdaten für Zusatzmodi, beispielsweise das Treppabgehen, das Fahrradfahren oder das Sitzen abgelegt. Diese Daten für die so genannten Zusatzmodi können über die Fernbedienung 6 eingestellt werden. Der Umfang der Einstellung richtet sich dabei unter anderem danach, wer der Nutzer der Fernbedienung 6 ist. Der Prothesennutzer wird mit eingeschränkten Rechten im Verhältnis zu einem Orthopädietechniker ausgestattet sein und nur in gewissen Grenzen verschiedene Parameter der Widerstandsänderungen verändern können. Beispielsweise können der Grad der Flexionsdämpfung in der Schwungphase an die individuellen Bedürfnisse angepasst werden. Ebenfalls ist es möglich, individuell Zusatzmodi zu deaktivieren oder zu aktivieren, wobei es möglich ist, die Zusatzmodi zu aktivieren, so dass sie gegenwärtig ausgeführt werden, ebenfalls ist es möglich, über die Fernbedienung 6 die Zusatzmodi freizuschalten, also die grundsätzliche Möglichkeit zur Aktivierung bereitzustellen, so dass nach Vorliegen entsprechender Sensordaten dann von der Steuerung automatisch das jeweilige Programm aktiviert wird.

Von der Steuerungseinrichtung in dem Unterschenkelteil 2 können die relevanten Daten der jeweiligen Fernbedienung 6 zugeordnet werden, so dass beispielsweise über grafische Symbole, so genannte Icons, eine leichte und benutzerfreundliche Zuordnung zu dem jeweiligen Gelenk und der jeweiligen Steuerung erfolgen kann. Die Fernbedienung 6 kann mehrere Gelenke einer Prothese versorgen, ebenfalls ist es möglich, dass über eine Fernbedienung 6 mehrere, ggf. unterschiedliche Prothesen mit mehreren, ggf. unterschiedlichen Gelenken und damit auch mit mehreren Steuereinrichtungen einzeln oder insgesamt versorgt werden, so dass ein Orthopädietechniker mit einer Fernbedienung 6 mehrere Patienten versorgen kann, so dass beispielsweise während des Gehens Parameter durch den Orthopädietechniker verändert werden. Es ist auch vorgesehen, dass ein Patient mit einer Fernbedienung 6 mehrere, ggf. unterschiedliche orthopädietechnische Einrichtungen einstellen kann. Dabei sind die Rechte des Orthopädietechnikers weitgehender als die des Prothesennutzers, da der Orthopädietechniker in der Regel über eine größere Kenntnis über die Zusammenhänge und die Abstimmung der Widerstandseinrichtung für die Orthese oder Prothese verfügt.

## Patentansprüche

1. System mit zumindest einer orthopädietechnischen Einrichtung mit zumindest einem Gelenk (4) und einer dem jeweiligen Gelenk (4) zugeordneten Widerstandseinrichtung, der eine Steuerungseinrichtung und ein Aktuator zugeordnet sind, wobei über den Aktuator das Gelenk bewegt oder der Widerstand gegen eine Beuge- und/oder Streckbewegung veränderbar ist, sowie mit einer Fernbedienung (6), die mit der Steuerungseinrichtung gekoppelt und über die das Widerstandsverhalten veränderbar ist, **dadurch gekennzeichnet, dass** in der orthopädietechnischen Einrichtung Daten für die Fernbedienung (6) abgelegt sind, mit denen die Fernbedienung (6) konfigurierbar ist und die über einen Pairing-Prozess auf die Fernbedienung (6) übertragen werden.

2. System nach Anspruch 1, **dadurch gekennzeichnet, dass** in der orthopädietechnischen Einrichtung ein Sender und in der Fernbedienung (6) ein Empfänger vorgesehen ist.

3. System nach Anspruch 1 oder 2, **dadurch gekennzeichnet, dass** in der Steuerungseinrichtung verschiedene Steuerungsmodi abgelegt sind, die über die Fernbedienung (6) aktivierbar und deaktivierbar sind.

4. System nach einem der voranstehenden Ansprüche, **dadurch gekennzeichnet, dass** in der orthopädietechnischen Einrichtung Daten gespeichert sind, die Steuerungsparameter, Steuerungsmodi, Sprache, Anordnung von Schaltflächen, Tastenbelegungen und /oder Einstellumfänge betreffen.

5. System nach einem der voranstehenden Ansprüche, **dadurch gekennzeichnet, dass** die Fernbedienung (6) ausgebildet ist, mehrere Steuerungseinrichtungen oder mehrere Gelenke anzusprechen.

6. System nach einem der voranstehenden Ansprüche, **dadurch gekennzeichnet, dass** der Nutzer der orthopädietechnischen Einrichtung gegenüber einem Orthopädietechniker eingeschränkte Rechte und Verstellmöglichkeiten hat.

7. System nach einem der voranstehenden Ansprüche, **dadurch gekennzeichnet, dass** die Fernbedienung (6) zur Umschaltung zwischen mehreren Gelenken oder Steuerungseinrichtungen für die Gelenke ausgebildet ist.

8. System nach einem der voranstehenden Ansprüche, **dadurch gekennzeichnet, dass** die Fernbedienung (6) ausgebildet ist, zwischen mehreren Steuerungsmodi umzuschalten und/oder Funktionen freizuschalten.

9. System nach einem der voranstehenden Ansprüche, **dadurch gekennzeichnet, dass** über die Fernbedienung (6) die Steuerungseinrichtung in einen Tiefschlafmodus schaltbar und/oder aus diesem herausholbar ist.

10. System nach einem der voranstehenden Ansprüche, **dadurch gekennzeichnet, dass** der Fernbedienung (6) eine Nutzerkennung zugeordnet ist, über die definiert ist, welche Parameter und/oder in welchem Umfang Parameter nutzerabhängig verstellbar sind.

11. System nach Anspruch 10, **dadurch gekennzeichnet, dass** die Nutzerkennung als eingebbarer Code ausgebildet ist.

12. System nach Anspruch 10 oder 11, **dadurch gekennzeichnet, dass** die Fernbedienung (6) eine Erfassungseinrichtung für biometrische Daten aufweist, über die eine Nutzerkennung zugeordnet wird.

## Claims

1. A system comprising at least one orthopedic device having at least one joint (4) and a resistance device associated with the respective joint (4), a control device and an actuator being associated with said resistance device, wherein the joint is moved or the resistance to a bending and/or stretching movement can be adjusted via the actuator, and comprising a remote control unit (6), which is coupled to the control device and via which the resistance properties can be adjusted, **characterized in that** data for the remote control unit (6) are stored in the orthopedic device, and said data can be used to configure the remote control unit (6) and are transmitted to the remote control unit (6) via a pairing process.

2. The system as claimed in claim 1, **characterized in that** a transmitter is provided in the orthopedic device and a receiver is provided in the remote control unit (6).

3. The system as claimed in claim 1 or 2, **characterized in that** various control modes are stored in the control device, it being possible for said control modes to be activated and deactivated via the remote control unit (6).

4. The system as claimed in one of the preceding claims, **characterized in that** data relating to the control parameters, control modes, language, arrangement of buttons, key assignments and/or adjustment ranges are stored in the orthopedic device.

5. The system as claimed in one of the preceding claims, **characterized in that** the remote control unit (6) is designed to address a plurality of control devices or a plurality of joints.

6. The system as claimed in one of the preceding claims, **characterized in that** the user of the orthopedic device has restricted rights and adjustment possibilities in comparison with an orthopedic technician.

7. The system as claimed in one of the preceding claims, **characterized in that** the remote control unit (6) is designed for switching over between a plurality of joints or control devices for the joints.

8. The system as claimed in one of the preceding claims, **characterized in that** the remote control unit (6) is designed to switch over between a plurality of control modes and/or enable functions.

9. The system as claimed in one of the preceding claims, **characterized in that** the control device can be switched into and/or withdrawn from a deep-sleep mode via the remote control unit (6).

10. The system as claimed in one of the preceding claims, **characterized in that** a user identification is assigned to the remote control unit (6) and is used to define which parameters and/or to what extent parameters can be adjusted in user-dependent fashion.

11. The system as claimed in claim 10, **characterized in that** the user identification is in the form of a code which can be input.

12. The system as claimed in claim 10 or 11, **characterized in that** the remote control unit (6) has a detection device for biometric data, via which a user identification is assigned.

## Revendications

1. Système comprenant au moins un dispositif technique orthopédique avec au moins une articulation (4) est un dispositif à résistance associé à l'articulation respective (4), dispositif auquel sont associés un moyen de commande et un actionneur, tels que l'articulation est déplacée ou la résistance à l'encontre d'un mouvement de flexion et/ou d'extension est variable au moyen de l'actionneur, et comprenant une télécommande (6), qui est couplée avec le moyen de commande et au moyen de laquelle le comportement de résistance peut être varié, **caractérisé en ce que** des données pour la télécommande (6) sont déposées dans le dispositif technique orthopédique, au moyen desquelles la télécommande (6) peut être configurée, et qui sont transmises à la télécommande (6) via un processus de pairage.

2. Système selon la revendication 1, **caractérisé en ce qu'**il est prévu un émetteur dans le dispositif technique orthopédique, et un récepteur dans la télécommande (6).

3. Système selon la revendication 1 ou 2, **caractérisé en ce que** différents modes de commande sont mémorisés dans le dispositif de commande, qui peuvent être activés et désactivés via la télécommande (6).

4. Système selon l'une des revendications précédentes, **caractérisé en ce que** dans le dispositif technique orthopédique sont mémorisées des données qui concernent des paramètres de commande, des modes de commande, la langue, l'agencement de surface de commutation, l'affectation des touches et/ou l'étendue des réglages.

5. Système selon l'une des revendications précédentes, **caractérisé en ce que** la télécommande (6) est réalisée pour s'adresser à plusieurs dispositifs de commande ou plusieurs articulations.

6. Système selon l'une des revendications précédentes, **caractérisé en ce que** l'utilisateur du dispositif technique orthopédique possède des droits et des possibilités de réglage restreint(e)s par rapport à un technicien orthopédiste.

7. Système selon l'une des revendications précédentes, **caractérisé en ce que** la télécommande (6) est réalisée pour commuter entre plusieurs articulations ou plusieurs dispositifs de commande pour les articulations.

8. Système selon l'une des revendications précédentes, **caractérisé en ce que** la télécommande (6) est réalisée pour commuter entre plusieurs modes de commande et/ou pour libérer des fonctions.

9. Système selon l'une des revendications précédentes, **caractérisé en ce que** le dispositif de commande est susceptible d'être commuté vers un mode de sommeil profond et/ou d'être sorti de celui-ci via la télécommande (6).

10. Système selon l'une des revendications précédentes, **caractérisé en ce qu'**une identification d'utilisateur est associée à la télécommande (6), identification via laquelle on définit quels paramètres et/ou dans quelle mesure des paramètres peuvent être modifiés en dépendance de l'utilisateur.

11. Système selon la revendication 10, **caractérisé en ce que** l'identification d'utilisateur est conçue sous la forme d'un code à saisir.

12. Système selon la revendication 10 ou 11, **caractérisé en ce que** la télécommande (6) comprend un moyen de saisie pour des données biométriques via lesquelles une identification d'utilisateur est associée.
